(19) Europäisches Patentamt
European Patent Office
Office européen des brevets

(11) Publication number: **0 141 593**
**A2**

# (12) EUROPEAN PATENT APPLICATION

(21) Application number: **84307199.4**

(22) Date of filing: **19.10.84**

(51) Int. Cl.⁴: **A 61 K 7/08**

(30) Priority: **21.10.83 US 544314**
**17.08.84 US 642723**
**25.01.84 US 573784**

(43) Date of publication of application: **15.05.85**
**Bulletin 85/20**

(84) Designated Contracting States: **AT BE CH DE FR GB IT LI NL SE**

(71) Applicant: **THE PROCTER & GAMBLE COMPANY,**
**301 East Sixth Street, Cincinnati Ohio 45201 (US)**

(72) Inventor: **Shulman, Joel Ira, 9427 Galecrest Drive, Cincinnati Ohio 45231 (US)**
Inventor: **Back, John Irvin, 5688 Cochran Road, Morrow Ohio 45152 (US)**

(74) Representative: **Brooks, Maxim Courtney et al, Procter & Gamble (NTC) Limited Whitley Road Longbenton,**
***Newcastle-upon-Tyne NE12 9TS (GB)***

## (54) Shampoo compositions.

(57) Shampoo compositions are disclosed containing a cationic conditioner compound, a zwitterionic surfactant, an ethoxylated amide and having a pH below about 3.75.

EP 0 141 593 A2

SHAMPOO COMPOSITIONS

JOEL I. SHULMAN
JOHN I. BACK

## BACKGROUND ART

The desire to develop products which simultaneously clean and condition hair has long been present. While the desire has been present, developing such products has presented innumerable problems. Generally the agents which condition hair best are cationic with one or more long fatty hydrocarbon chains. Hair being negatively charged will allow for the cationic portion to attach to the hair while the long fatty chain(s) provide for ease of combing and hair conditioning.

Cationic materials generally cannot be used with good cleaning anionic surfactants and still deliver good hair condition. This meant that other surfactants such as nonionics, amphoterics and zwitterionics were examined by workers in the field. Many of these efforts are reflected in patents issued in the conditioning shampoo area.

U.S. Patent 3,849,348, November 19, 1974 to Hewitt discloses conditioning shampoos containing betaine, cationic and amine oxide surfactants. U.S. Patent 3,697,452, October 10, 1972 to Olson et al discloses shampoo compositions similar to those in Hewitt. Another patent to Hewitt is U.S. Patent 3,755,559, August 28, 1973 disclosing shampoos containing a tertiary amine oxide, a higher alkyl betaine and a soap. U.S. Patent 3,822,312, July 2, 1974 to Sato discloses shampoos containing a quaternary ammonium salt, a betaine and an additional additive. U.S. Patent 3,990,991, November 9, 1976 to Gerstein discloses shampoos containing amphoteric surfactants and quaternary ammonium compounds. U.S. Patent 4,080,310, March 21, 1978 to Ng et al discloses shampoos containing an amphoteric surfactant, a cationic resin and having a pH as low as 3. U.S. Patent 4,132,679, January 2, 1979 to Tsutsumi et al discloses shampoos containing a

phosphoric acid ester salt and a betaine. U.S. Patent 4,231,903, November 4, 1980 to Lindemann et al discloses shampoos containing a mixture of an amido betaine and a phosphobetaine. U.S. Patent 4,247,548, January 27, 1981 to Barker discloses a conditioning shampoo containing a betaine, a polypropoxylated quaternary ammonium chloride surfactant and gum arabic. U.S. Patent 4,294,728, October 13, 1981 to Vanlerberghe et al discloses shampoos containing a cationic, amphoteric or zwitterionic surfactant and a diol. U.S. Patent 4,329,335, May 11, 1981 to Su et al discloses a shampoo composition containing a betaine, an amine oxide and a polymerized quaternary compound. U.S. Patent 4,181,634, January 1, 1980 to Kennedy et al discloses shampoos containing a betaine and a bisquaternary compound.

Ethoxylated amides have been recognized for use in shampoos (see E. A. Kraggs, Soap and Chemical Specialties, 40 (1964):1, pg. 79.), but not in compositions of the type disclosed and claimed herein.

An additional problem faced by formulators of conditioning shampoos has been achieving formulations which possess good cleaning and conditioning over an extended period of time. This requires that the compositions degrade only minimally over time.

While the above described references disclose compositions containing components of the type used in the present compositions, they do not teach or suggest totally satisfactory answers to the questions of good cleaning, conditioning and stability. It is believed that good cleaning with cationic conditioning compounds is in part dependent on limiting the reacting of the quaternary with the fatty acids in sebum.

In addition the references fail to teach or suggest compositions of the type disclosed herein having a pH below 3.75, preferably in the range of from about 2 to about 3.75.

It is, therefore, an object of the present invention to provide hair conditioning shampoo compositions which provide good cleaning and conditioning. The good cleaning relates to improved sebum emulsification as well as good lather.

It is a further object of the present invention to provide shampoo compositions which are stable in terms of providing good cleaning and conditioning over an extended period of time. It is believed that the ethoxylated amide's resistance to hydrolysis contributes to this improved stability.

It is still a further object of the present invention to provide compositions which can provide desired viscosities without resorting to additional components to the extent necessary if the preferred essential components are not used.

These and other objects will become more apparent from the detailed description which follows.

Unless otherwise indicated, all percentages and ratios herein are by weight.

## DISCLOSURE OF THE INVENTION

The compositions of the present invention comprise from about 5% to 70% of a zwitterionic surfactant, from about 0.5% to about 10% of a cationic conditioning compound, from about 1.0% to 10% of an ethoxylated amide, preferably the ethoxylated amide has an alkyl group of from 12 to 18 carbon atoms, from about 20% to about 93.5% water and have a pH below about 3.75.

## DETAILED DESCRIPTION OF THE INVENTION

The essential as well as optional components of the present invention are described in detail below.

### Surfactant

The essential surfactants used in the compositions of the present invention are higher alkylamido betaines.

The betaines may be represented by the following structural formula:

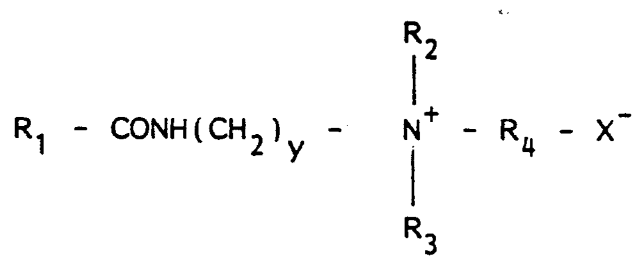

wherein $R_1$ is a long chain alkyl radical having from about 10 to about 18 carbon atoms, $R_2$ and $R_3$ are each alkyl radicals having from about 1 to about 3 carbon atoms, $R_4$ is an alkylene or hydroxy alkylene radical having from about 1 to about 4 carbon atoms, y is an integer from 1 to 4, and X is a carboxylate radical. $R_1$ may be a mixture of long chain alkyl radicals and may contain one or more intermediate linkages or non-functional substituents such as hydroxyl or halogen radicals which do not affect the hydrophobic character of the radical. Examples of betaines useful herein include cocamidopropyldimethylcarboxymethyl betaine, laurylamidopropyldimethylcarboxymethyl betaine among many others. In many instances the dimethylcarboxymethyl part of the designation is not included. Mixtures of the betaines are also acceptable.

The amount of surfactant is from about 5% to about 70%, preferably from about 10% to about 25%.

<u>Cationic Conditioning Compound</u>

The second essential component of the present invention is a cationic conditioning compound, preferably a quaternary ammonium salt, a imidazolinium salt or mixtures of one or both.

Quaternary ammonium salts can have the formula:

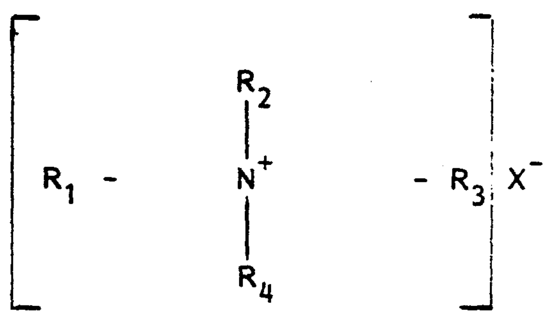

wherein $R_1$ is hydrogen, an aliphatic group of from about 1 to 22 carbon atoms, or an aromatic, aryl or alkaryl group having from about 6 to 20 carbon atoms; $R_2$ is an aliphatic group having from about 12 to 22 carbon atoms; $R_3$ and $R_4$ are each alkyl groups having from 1 to 3 carbon atoms; and X is an anion selected from halogen, acetate, phosphate, nitrate and methyl sulfate radicals.

Preferred quaternary ammonium salts are the dialkyldimethylammonium chlorides, wherein the alkyl groups have from 12 to 22 carbon atoms and are derived from long chain fatty

acids, such as tallow or hydrogenated tallow. The term "tallow" refers to fatty alkyl groups derived from tallow fatty acids. Such fatty acids give rise to quaternary compounds where $R_1$ and $R_2$ have predominately from 16 to 18 carbon atoms.

Representative examples of quaternary ammonium salts useful in this invention include ditallowdimethylammonium chloride, ditallowdimethylammonium methyl sulfate, dihexadecyldimethylammonium chloride, di(hydrogenated tallow) dimethylammonium chloride, dioctadecyldimethylammonium chloride, dieicosyldimethylammonium chloride, didocosyldimethylammonium chloride, di(hydrogenated tallow)dimethylammonium acetate, dihexadecyldiethylammonium chloride, dihexadecyldimethylammonium acetate, ditallowdipropylammonium phosphate, ditallowdimethylammonium nitrate, di(coconut-alkyl)dimethylammonium chloride; cetyltrimethylammonium chloride; stearyldimethylbenzylammonium chloride, and myristylalkonium chloride.

Other quaternary ammonium salts useful herein are the compounds of the formula

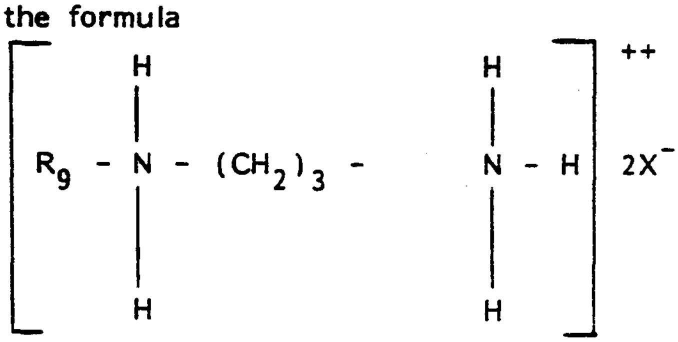

wherein $R_9$ is an aliphatic group having 16 to 22 carbon atoms and X is an anion as above defined. Tallow propanediamine hydrochloride is an example of this quaternary ammonium salt.

Quaternary imidazolinium salts have the formula

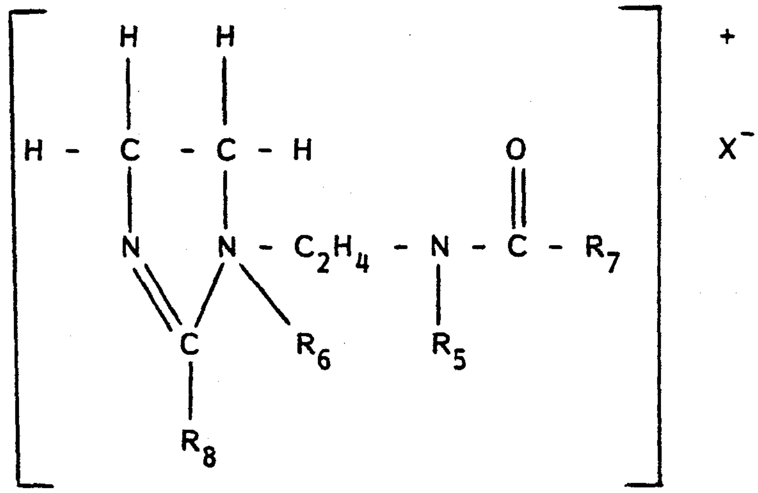

wherein $R_6$ is an alkyl group containing from 1 to 4, preferably from 1 to 2 carbon atoms; $R_5$ is an alkyl group containing from 1 to 4 carbon atoms or a hydrogen atom; $R_8$ is an alkyl group containing from 1 to 22, preferably at least 15 carbon atoms, or a hydrogen atom; $R_7$ is an alkyl group containing from 8 to 22, preferably at least 15, carbon atoms; and X is an anion, preferably chloride. Other suitable anions include those disclosed with reference to the quaternary ammonium salts described hereinbefore.

Particularly preferred are those imidazolinium salts in which both $R_7$ and $R_8$ are alkyl of from 12 to 22 carbon atoms, e.g. 1-methyl-1-[(stearoylamide)ethyl]-2-heptadecyl-4,5-dihydroimidazolinium chloride; 1-methyl-1-[(palmitoylamide)ethyl]-2-octadecyl-4,5-dihydroimidazolinium chloride; and 1-methyl-1-[(tallowamide)--ethyl]-2-tallow-imidazolinium methyl sulfate.

Another imidazoline derivative favored for use in the present compositions is isostearyl ethylimidonium ethosulfate.

The cationic conditioning compound is present at a level of from about 0.5% to about 10%, preferably from about 1% to about 6%.

Ethoxylated Amide

The third essential component of the compositions described herein is an ethoxylated amide. These are generally ethoxylated mono- and diethanolamides of fatty acids having from about 8 to about 18 carbon atoms, preferred are alkyl groups having 12 to 18

carbon atoms. It is also preferred that the amides have only a small percentage, less than about 3%, of alkyl groups at or below $C_{10}$ in length. The number of ethoxy groups ($CH_2CH_2O$) present in addition to that present in the case of the mono material is from 1 to about 5 while in the case of the di material it is from about 2 to about 4. Such products are commonly identified as PEG-X-alkylamide such as PEG-3 cocamide or PEG-6 lauramide, preferred materials for use herein. Other suitable alkyl groups include myristyl, cetyl and tallow. Mixtures of amides may also be used. A preferred mixture is a 70:30 mix of amides having lauryl and myristyl alkyl groups.

pH Adjustment

The compositions of the present invention have a pH below about 3.75, preferably in the range of from about 2.0 to about 3.75. The compositions are adjusted to this pH range with an acid buffer. The buffering capability is necessary since the pH of the diluted product (approximately 10:1 with water) when applied to hair should be near the range given. Suitable buffer solutions can be prepared using for example, agents such as citric acid, phosphoric acid, phthalic acid, glycine or mixtures thereof. In each case the proper buffering capacity is obtained by adjusting the final pH of the compositions to within the pH range indicated above. This may be done by using a strong acid or a strong base (e.g., HCl or NaOH) as may be needed. The most preferred agent is citric acid. The amount of buffer employed in the present compositions depends on the particular acid chosen but is generally from about 0.3% to about 10%, preferably from about 1% to about 5%.

Water

Water is the last essential component of the present invention and forms the remainder of the composition. It is generally present at a level of from about 20% to about 93.5% preferably from about 65% to about 80%.

Optional Components

The shampoos herein can contain a variety of non-essential optional components suitable for rendering such compositions more stable and desirable. Such conventional optional ingredients are well known to those skilled in the art, e.g., preservatives such as benzyl alcohol, methyl paraben, propyl paraben and imidazolidinyl urea; betaine surfactants such as lauryl betaine in an amount up to about equal to the amount of the amidobetaine; thickeners and viscosity modifiers such as, sodium chloride, sodium sulfate, $C_{10}$ - $C_{14}$ alkyl alcohols (e.g. lauryl alcohol), and ethyl alcohol; suspending agents such as hydrogenated castor oil; opacifiers such as ethylene glycol distearate; perfumes; dyes; antidandruff agents such as zinc pyridinethione; other conditioning agents such as amino functional silicones; and sequestering agents such as disodium ethylenediamine tetraacetate. Such agents, except for the betaine surfactants, generally are used individually at a level of from about 0.01% to about 10%.

METHOD OF MANUFACTURE

The shampoos of the present invention may be made in a variety of ways. A preferred method is set forth in Example I.

INDUSTRIAL APPLICABILITY

The present compositions are used in a conventional manner for cleaning hair. From about 0.1g to about 10g of the composition is applied to hair that has been wetted, generally with water, worked through the hair and then rinsed out.

The following Examples further describe and demonstrate the preferred embodiments within the scope of the present invention. The Examples are given solely for the purpose of illustration and are not to be construed as limitations of the present invention as many variations thereof are possible without departing from its spirit and scope.

## EXAMPLE I

The following composition representative of the present invention was prepared:

| COMPONENT | WEIGHT % |
|---|---|
| Lexaine LM[1] | 18.0 |
| Citric Acid | 3.5 |
| Adogen 470DE[2] | 4.0 |
| Crotein Q[3] | 1.0 |
| Mazamide C-2[4] | 3.0 |
| Lauryl Alcohol | 0.4 |
| Ethylene Glycol Distearate | 1.5 |
| Sodium Chloride | 1.0 |
| Color, Perfume, Preservative and Water | q.s. 100% |

[1] Lauramidopropyl betaine - Inolex Chemical Co.
[2] Di-(partially hydrogenated) tallow dimethylammonium chloride - Sherex Chemical Co.
[3] Steartrimonium hydrolyzed animal protein - Croda, Inc.
[4] PEG-3 Cocamide - Mazer Chemical Company

Manufacturing Method for Example I

Lexaine LM and water are combined and heated to 160°F under constant agitation. In a separate vessel, the protein compound is dissolved in room temperature water to make a 20% active solution. The ethoxylated amide, Ethylene Glycol Distearate, Adogen 470DE, lauryl alcohol, citric acid and sodium chloride are added in this sequence to the Lexaine LM water mix while the temperature is maintained. Once an essentially clear, homogeneous mixture is obtained, the composition is cooled to 120°F. At this temperature, the remaining ingredients, including the protein premix, and any make-up water are added to achieve the desired composition. The mixture is subsequently cooled to room temperature.

This composition possessed good stability, cleaning performance and conditioning performance.

EXAMPLE II

The following composition representative of the present invention was prepared:

| COMPONENT | WEIGHT % |
|---|---|
| Lexaine LM[1] | 18.0 |
| Citric Acid | 3.5 |
| Adogen 470DE[2] | 2.0 |
| Amino Functional Silicone[5] | 1.75 |
| Ethoxylated Amide[3] | 4.5 |
| Variquat E-228[4] | 2.0 |
| Lauryl Alcohol | 0.5 |
| Ethylene Glycol Distearate | 1.5 |
| Sodium Chloride | 4.0 |
| Color, Perfume, Preservative and Water q.s. | 100% |

[1] Lauramidopropyl betaine - Inolex Chemical Co.

[2] Di-(partially) hydrogenated tallow dimethylammonium chloride - Sherex Chemical Co.

[3] PEG-3 Alkyl (98% $C_{12}$) Amide (Mazamide C-2 Experimental) - Mazer Chemical Company

[4] Cetrimonium chloride - Sherex Chemical Company

[5] DC Q2 - 8075 offered by Dow Corning Corporation

A similar composition was prepared but using PEG-3-Cocamide rather than PEG-3 Lauramide (the difference being that the latter was 98% $C_{12}$ while the former had only 48% with the amount $C_{10}$ or shorter being in excess of 15%). When the compositions were cycled between 0°F, 24 hours, and 50°F, 24 hours, the composition containing the cocamide showed phase separation after 2 cycles while the lauramide did not show separation even after 4 cycles. A sample of each composition was stored at 80°F for 24 hours after each cycle before the stability determination was made.

EXAMPLE III

The following composition was also prepared:

| COMPONENT | | WEIGHT % |
|---|---|---|
| Lexaine LM | | 18.0 |
| Citric Acid | | 3.5 |
| Adogen 470DE | | 2.0 |
| Variquat E-228[1] | | 2.0 |
| Mazamide C-2 | | 4.5 |
| Lauryl Alcohol | | 0.5 |
| Ethylene glycol Distearate | | 1.5 |
| Sodium Chloride | | 1.0 |
| Preservative, perfume, color and water | q.s. | 100% |

[1]Cetrimonium chloride - Sherex Chemical Co.

This composition performed well in the areas of cleaning, conditioning and stability.

EXAMPLE IV

The following composition representative of the present invention using a preferred amide mixture was prepared:

| COMPONENT | | WEIGHT % |
|---|---|---|
| Lexaine LM | | 18.0 |
| Ethoxylated Amide[1] | | 4.5 |
| Ethylene Glycol Distearate | | 1.5 |
| Lauryl Alcohol | | 0.5 |
| Adogen 470DE | | 2.0 |
| Variquat E-228 | | 2.0 |
| Citric Acid | | 3.5 |
| Color, Perfume, Preservative and Water | q.s. | 100% |

[1]PEG-3 Alkyl (70% $C_{12}$, 30% $C_{14}$) Amide (Mazamide C-2 Experimental) - Mazer Chemical Company

EXAMPLE V

The following composition was also prepared:

| COMPONENT | | WEIGHT % |
|---|---|---|
| Lexaine LM | | 18.0 |
| Citric Acid | | 3.5 |
| Adogen 470DE | | 2.0 |
| Variquat E-228 | | 2.0 |
| Mazamide C-5[1] | | 4.5 |
| Lauryl Alcohol | | 2.0 |
| Ethylene glycol Distearate | | 1.5 |
| Sodium Chloride | | 1.5 |
| Preservative, perfume, color and water | q.s. | 100% |

[1] PEG-6 Cocamide - Mazer Chemical Company.

EXAMPLE VI

The following preferred composition of the present invention was also prepared:

| COMPONENT | | WEIGHT % |
|---|---|---|
| Lexaine LM | | 18.0 |
| Ethoxylated Amide as in Example II | | 4.5 |
| Ethylene Glycol Distearate | | 1.5 |
| Lauryl Alcohol | | 0.5 |
| Adogen 470DE | | 2.0 |
| Variquat E-228 | | 2.0 |
| Citric Acid | | 3.5 |
| Preservative, Color, Perfume and water | q.s. | 100% |

EXAMPLE VII

The following composition of the present invention was prepared:

| COMPONENT | | WEIGHT % |
|---|---|---|
| Lexaine LM | | 18.0 |
| Ethoxylated Amide as in Example II | | 4.5 |
| Variquat E-228 | | 4.0 |
| Lauryl Alcohol | | 0.5 |
| Citric Acid | | 3.5 |
| Preservative, Color, Perfume and Water | q.s. | 100% |

EXAMPLE VIII

The following composition was also prepared:

| COMPONENT | | WEIGHT % |
|---|---|---|
| Lexaine LM | | 18.00 |
| Citric Acid | | 3.50 |
| Adogen 470DE | | 4.05 |
| Mazamide C-5[1] | | 4.50 |
| Crotein Q | | 5.00 |
| Lauryl Alcohol | | 2.00 |
| Ethylene glycol Distearate | | 1.50 |
| Sodium Chloride | | 2.00 |
| Color, preservative, perfume and water | q.s. | 100% |

[1] PEG-6 Cocamide - Mazer Chemical Company.

WHAT IS CLAIMED IS:

Claims

1. A shampoo composition characterised by:
   (a) from 0.5% to 10% of one or more cationic hair conditioning agents;
   (b) from 5% to 70% of one or more higher alkylamido betaines;
   (c) from 1.0% to 10% of one or more mono or diethoxylated amides; and
   (d) water;

   wherein the total number of ethoxy groups in the case of the mono amide material is from 2 to 6 and in the case of the di material is from 4 to 6 and the pH of said composition is less than 3.75, being maintained within said range by means of a buffering agent.

2. A shampoo composition according to Claim 1 wherein said ethoxylated amides have alkyl groups of from 12 to 18 carbon atoms with less than 3% of the groups being 10 carbon atoms long or shorter.

3. A shampoo composition according to Claim 2 wherein said ethoxylated amide is selected from PEG-3 lauramide, PEG-3 myristamide and mixtures thereof.

4. A shampoo composition according to Claim 1 wherein said ethoxylated amide is selected from PEG-3 cocamide, PEG-6 lauramide and mixtures thereof.

5. A shampoo composition according to any of Claims 1 to 4 wherein the cationic hair conditioning agent is selected from quaternary ammonium compounds, quaternary imidazolinium compounds and mixtures thereof.

6. A shampoo composition according to any of Claims 1 to 5 wherein the pH is from 2.0 to 3.75.

7. A shampoo composition according to any of Claims 1 to 6 wherein said pH adjusting acid is citric acid, said cationic hair conditioning agent is ditallowdimethyl ammonium chloride and said alkylamido betaine is cocoamidopropyl betaine.

8. A method of cleaning hair comprising:
   (a) applying from 0.1g to 10g of a composition according to any of Claims 1 to 7 to hair that has been wetted;
   (b) working said composition through said hair; and
   (c) rinsing said composition from said hair.